# FASCICULE DE BREVET EUROPEEN

(11) **EP 1 679 570 B1**
(45) Date de publication et mention de la délivrance du brevet: **23.07.2008**
(21) Numéro de dépôt: 05300935.3
(22) Date de dépôt: 17.11.2005
(51) Int. Cl.: G05D 16/20

(54) **Dispositif régulateur de vide à sélecteur de pression utilisable en milieu hospitalier**
In Krankenhausumgebungen verwendbare Vakuumreglervorrichtung mit Druckwahlschalter
Vacuum regulator device with pressure gauge for use in hospitals

(30) Priorité: 06.01.2005 FR 0550046
(43) Date de publication de la demande: 12.07.2006
(73) Titulaire: TAEMA, F-92182 Antony Cédex (FR)
(72) Inventeur: Collado, Pedro, 77330 Ozoir La Ferriere (FR); Rudnianyn, Philippe, 91310 Longpont sur Orge (FR); Regnier, Guillaume, 91620 Nozay (FR)
(74) Mandataire: De Cuenca, Emmanuel Jaime

(56) Documents cités:
- WO-A-00/56378
- US-A- 4 795 448
- US-A- 5 224 649

## Description

L'invention concerne un dispositif de régulation de dépression ou de vide, en particulier utilisable dans le domaine médical, comportant des moyens de sélection du niveau de vide désiré.

Habituellement, les dispositifs de régulation du niveau de vide, encore appelés régulateurs de dépression, venant se connecter sur les prises murales des réseaux de vide au sein des bâtiments hospitaliers ou similaires, sont équipés de moyens permettant l'ouverture ou la fermeture directe de l'alimentation ou de l'arrivée de vide ; la lecture de la dépression (vide), via un vacuomètre indiquant les dépressions de 0 à 1000 mbar ; et la régulation de la pression de vide, typiquement entre 0 et 250 mbar pour certaines applications et entre 0 et 600 mbar pour d'autres applications.

Lorsque le réglage de la pression (c'est-à-dire du niveau de vide souhaité) s'effectue en continu par rotation d'un bouton rotatif de réglage du dispositif régulateur de vide, l'opérateur est simultanément obligé de contrôler visuellement la pression affichée sur le vacuomètre et d'ajuster alors précisément le niveau pression en tournant le bouton de réglage, dans un sens ou dans l'autre, pour augmenter ou diminuer le vide qui en résulte jusqu'à la valeur désirée.

Ce type de réglage est appelé 'système de réglage linéaire' car, avec ce système de réglage, on peut afficher toutes les pressions sur la plage de pression concernée du vacuomètre.

Ce genre de système de réglage est cependant très peu pratique à mettre en oeuvre puisqu'il nécessite d'opérer un réglage 'indirect' du niveau de vide, c'est-à-dire en se basant sur une indication donnée par le cadran d'un vacuomètre incorporé au dispositif de réglage.

Par ailleurs, il présente un autre inconvénient majeur, à savoir que le médecin ayant requis un niveau de vide donné, n'a pas l'assurance que l'opérateur opérant le réglage, par exemple une infirmière, respecte strictement sa consigne.

De plus, on comprend que le fait de devoir se baser sur l'afficheur à aiguille du vacuomètre pour régler le niveau de vide peut engendrer un mauvais réglage, par exemple du fait d'un défaut de parallaxe ou analogue.

Le problème qui se pose est alors d'améliorer les dispositifs existants et de proposer un dispositif de réglage simple, qui ne nécessite pas obligatoirement la présence d'un vacuomètre pour contrôler le réglage que l'on vient d'effectuer et qui permette de s'assurer que le niveau de vide demandé par le médecin est bien celui qui a été sélectionné par l'opérateur.

Autrement dit, le but de l'invention est d'améliorer les dispositifs de régulation de vide connus de manière à aboutir à un système régulateur de vide d'utilisation simple et rapide, c'est-à-dire sans tâtonnement de la part de l'opérateur lors de l'étape de réglage de la plage de pression souhaitée.

Le document US-A-4 795 448 décrit un dispositif régulateur de vide conforme au préambule de la revendication 1.

La solution de l'invention est un dispositif régulateur de vide dans lequel : chaque position angulaire est indexée et marquée par un repère ou un marquage répartis autour de l'axe de rotation du moyen sélecteur de manière à indiquer directement à l'opérateur le niveau de vide correspondant à la position angulaire dans laquelle est positionné le moyen sélecteur à un instant donné, et en ce que le sélecteur rotatif ou, respectivement, le corps du dispositif ou une structure solidaire dudit corps, comprend une pièce mobile normalement repoussée par un moyen à ressort et en ce que, dans chacune des positions angulaires indexées, ladite pièce mobile vient s'insérer, au moins partiellement, dans un évidement aménagé dans le corps du dispositif ou dans une structure solidaire dudit corps, respectivement dans au moins un évidemment aménagé dans le sélecteur rotatif .

Selon le cas, le dispositif de l'invention peut comprendre l'une ou plusieurs des caractéristiques suivantes :
- le sélecteur de niveau de vide comporte un moyen d'indication de position (permettant d'indiquer le positionnement angulaire du sélecteur autour de son axe X-X et par rapport aux repères ou marquages.
- le moyen d'indication de position est une marque portée par le sélecteur rotatif ou une fenêtre de lecture aménagée dans le sélecteur rotatif.
- le sélecteur rotatif est en polymère.
- les moyens internes de réglage de vide comprenant au moins un siège et un clapet et en ce que le déplacement du sélecteur d'une position angulaire indexée à une autre position angulaire indexée P1, P2, ...Pn, engendre une modification du positionnement du clapet par rapport audit siège.
- le déplacement maximal en rotation du sélecteur se fait selon un angle inférieur à 360° autour de l'axe X-X.
- les repères ou marquages répartis autour de l'axe de rotation X-X du moyen sélecteur sont portés par une structure formant un capotage externe, de préférence un capotage en polymère.
- les repères ou marquages sont situés un cadran agencé ou fixé autour de l'axe X-X, de préférence ledit cadran est situé sur la structure formant capotage externe.

Grâce à l'invention, le coût et la fiabilité des appareils de régulation de vide sont améliorés puisque l'opérateur peut afficher directement le niveau de pression choisi, par sélection immédiate de celui-ci en opérant un positionnement direct du sélecteur de niveau de vide dans une position angulaire indexée correspondant au niveau de vide qu'il souhaite obtenir, donc sans nécessité d'utiliser un vacuomètre, lequel peut alors être supprimé.

L'invention va être mieux comprise grâce à la description détaillée donnée ci-après à titre illustratif, en liaison avec les figures annexées.

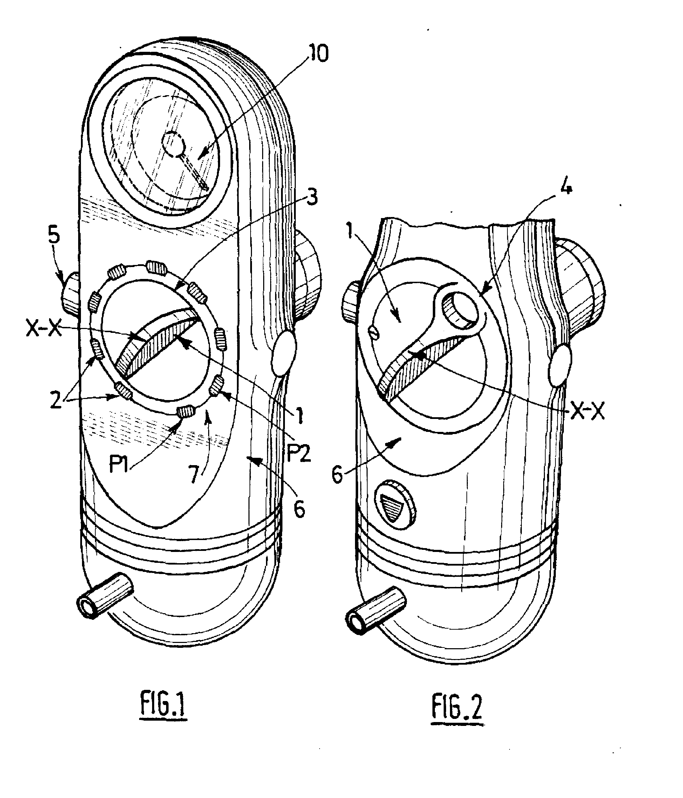

Les Figures 1 et 2 représentent deux modes de réalisation d'un dispositif régulateur de vide selon l'invention, lequel est destiné à être relié ou connecté à un réseau de distribution de vide, par exemple dans un bâtiment hospitalier, grâce à un raccord normalisé.

La régulation du niveau de vide se fait par un ensemble interne de régulation de vide classique comprenant un siège, un clapet, une membrane ou un piston, un ressort de détente et une vis de réglage de la pression. De tels moyens de régulation de niveau de vide (ou de dépression) sont identiques à ceux utilisés dans l'art antérieur et ne seront donc pas détaillés davantage ici.

De plus, le dispositif régulateur de vide comporte aussi, de manière également habituelle, un filtre anti-retour et un bocal de sécurité situé à sa base et destiné à recueillir les déchets ou liquides aspirés par le dispositif, lors de son utilisation pendant une opération de soins médicaux.

En outre, le dispositif possède un système d'arrêt de fluide (vide) commandé manuellement par un moyen de commande externe, par exemple de type à poussoir 5 à double sens.

Comme montré sur la Figure 1, sur le dispositif de régulation de vide de l'invention, le bouton de réglage de pression linéaire, classiquement utilisé sur les appareils de l'art antérieur, a été remplacé par un sélecteur 1 de pression indexable à plusieurs positions angulaires P1, P2...Pn, dont chaque position angulaire correspond à une valeur ou une plage de valeurs de pression préfixées.

Selon l'invention, les différentes valeurs ou plages de valeurs sont repérées par des marquages ou repères situés sur la périphérie du sélecteur 1 rotatif de réglage, c'est-à-dire autour de l'axe XX du sélecteur 1. De préférence, les marquages ou repères sont des valeurs de pression discontinues, par exemple 0, -30 mbar, -60 mbar, -100 mbar, -200 mbar, -300 mbar, -400 mbar, - 600 mbar... Avantageusement, les valeurs de pression sélectionnables correspondant aux dits repères ou marquages sont suffisamment proches les unes des autres pour permettre un réglage approprié dans une indication médicale donnée.

Le système de l'invention conduit donc non plus à un réglage linéaire de vide, comme selon l'art antérieur, mais à un réglage séquentiel ou discontinu du niveau de vide, selon des valeurs ou dans des plages de pressions préfixées et en nombre limité.

Ainsi, lorsque l'opérateur souhaitera obtenir un niveau de vide de -100 mbar par exemple, il n'aura qu'à positionner le bouton sélecteur 1 devant le repère de la position angulaire P4 sur la figure 1 correspondant à la valeur de pression voulue et ce, sans risque d'erreur possible puisque le niveau de vide correspondant à cette position P4 aura été pré-réglé en usine et ne sera pas modifiable par l'opérateur.

Afin de repérer la position angulaire du sélecteur 1 de vide, celui-ci est muni d'un repère de position 3, par exemple une marque comme montré en figure 1, ou alors il est également possible de faire apparaître la valeur de vide présélectionnée dans une fenêtre 4 ménagée dans le sélecteur 1 rotatif, comme illustré en figure 2.

Autrement dit, selon le mode de réalisation choisi, le bouton de sélection 1 possède soit une fenêtre 4 de lecture de la plage de vide sélectionnée, lorsque le bouton 1 de sélection recouvre les indications de niveau de vide (Fig. 2), soit un point de repérage 3 permettant de mettre en vis à vis les plages ou valeurs 2 de pressions à sélectionner et ledit point de repérage 3, lorsque ces plages de pression sont imprimées autour du bouton sélecteur 1 (Fig. 1).

Dans tous les cas, on comprend que, grâce à la présente invention, l'opérateur n'a plus à effectuer le réglage linéaire fin qui était nécessaire avec les dispositifs de l'art antérieur et que le contrôle parallèle sur un vacuomètre 10 devient également totalement inutile car les positions sont indexées.

## Revendications

1. Dispositif régulateur de vide comprenant :
- un moyen sélecteur (1) de niveau de vide, actionnable manuellement par l'opérateur, mobile en rotation autour d'un axe de rotation (X-X) et agissant sur des moyens internes de réglage de vide pour permettre de régler le niveau de vide souhaité, et
- des moyens indicateurs de niveau de vide (P1, P2...Pn ; 2) permettant d'indiquer à l'opérateur un niveau de vide à un instant donné,
le moyen sélecteur (1) étant apte à être déplacé par l'opérateur entre plusieurs positions (P1, P2...Pn) angulairement espacées les unes des autres, chaque position angulaire (P1, P2...Pn) correspondant à au moins une valeur de niveau de vide sélectionnable,
**caractérisé en ce que** chaque position angulaire (P1, P2...Pn) est indexée et marquée par un repère ou un marquage (2) répartis autour de l'axe de rotation (X-X) du moyen sélecteur (1) de manière à indiquer directement à l'opérateur le niveau de vide correspondant à la position angulaire (P1, P2...Pn) dans laquelle est positionné le moyen sélecteur (1) à un instant donné, et **en ce que** le sélecteur (1) rotatif ou, respectivement le corps du dispositif ou une structure solidaire dudit corps, comprend une pièce mobile normalement repoussée par un moyen à ressort et **en ce que**, dans chacune des positions angulaires indexées (P1, P2, ...Pn), ladite pièce mobile vient s'insérer, au moins partiellement, dans un évidement aménagé dans le corps du dispositif ou dans une structure solidaire dudit corps, respectivement dans au moins un évidement aménagé dans le sélecteur (1) rotatif.

2. Dispositif selon la revendication 1, **caractérisé en ce que** le sélecteur (1) de niveau de vide comporte un moyen d'indication de position (3, 4) permettant d'indiquer le positionnement angulaire du sélecteur (1) autour de son axe (X-X) et par rapport aux repères (2).

3. Dispositif selon la revendication 2, **caractérisé en ce que** le moyen d'indication de position (3, 4) est une marque (3) portée par le sélecteur (1) rotatif ou une fenêtre (4) de lecture aménagée dans le sélecteur (1) rotatif.

4. Dispositif selon l'une des revendications 1 à 3, **caractérisé en ce que** le sélecteur (1) rotatif est en polymère.

5. Dispositif selon l'une des revendications 1 à 4, **caractérisé en ce que** les moyens internes de réglage de vide comprenant au moins un siège et un clapet et **en ce que** le déplacement du sélecteur (1) d'une position angulaire indexée à une autre position angulaire indexée (P1, P2, ...Pn), engendre une modification du positionnement du clapet par rapport audit siège.

6. Dispositif selon l'une des revendications 1 à 5, **caractérisé en ce que** le déplacement maximal en rotation du sélecteur (1) se fait selon un angle inférieur à 360° autour de l'axe (X-X).

7. Dispositif selon l'une des revendications 1 à 6, **caractérisé en ce que** les repères ou marquages (2) répartis autour de l'axe de rotation (X-X) du moyen sélecteur (1) sont portés par une structure formant un capotage externe (6), de préférence un capotage en polymère.

8. Dispositif selon la revendication 7, **caractérisé en ce que** les repères ou marquages (2) sont situés un cadran (7) agencé ou fixé autour de l'axe (X-X), de préférence ledit cadran (7) est situé sur la structure formant capotage externe (6).

## Claims

1. Vacuum regulator device comprising:
- a selection means (1) for selecting the vacuum level, manually activated by the operator, rotatable about a rotation axis (X-X) and acting on internal means for regulating the vacuum in order to allow regulation of the desired vacuum level; and
- means (P1, P2 ... Pn; 2) of indicating the vacuum level enabling of the vacuum level at a given moment to be indicated to the operator;
the selection means (1) being able to be moved by the operator between several positions (P1, P2 ... Pn) angularly separated from each other, each angular position (P1, P2 ... Pn) corresponding to at least one selectable vacuum level value,
**characterized in that** each angular position (P1, P2 ... Pn) is indexed and marked by a reference point or a marking (2) distributed around the rotation axis (X-X) of the selection means (1) so as to indicate directly to the operator the vacuum level corresponding to the angular position (P1, P2 ... Pn) in which the selection means (1) is positioned at a given moment, and **in that** the rotating selector (1), or the body of the device or a structure joined to said body, respectively, comprises a movable piece normally depressed by a spring means, and **in that** in each of the indexed angular positions (P1, P2 ... Pn) said movable piece is inserted, at least partly, into a recess provided in the body of the device or in a structure joined to said body or into at least one recess provided in the rotating selector (1), respectively.

2. Device according to Claim 1, **characterized in that** the vacuum level selector (1) comprises a position indication means (3, 4) enabling indication of the angular positioning of the selector (1) about its axis (X-X) and in relation to the reference points (2).

3. Device according to Claim 2, **characterized in that** the position indication means (3, 4) is a mark (3) on the rotating selector (1) or a reading window (4) provided in the rotating selector (1).

4. Device according to one of Claims 1 to 3, **characterized in that** the rotating selector (1) is made of a polymer.

5. Device according to one of Claims 1 to 4, **characterized in that** the internal means for regulating the vacuum comprise at least a seat and a valve and **in that** the movement of the selector (1) from one indexed angular position to another indexed angular position (P1, P2 ... Pn) gives rise to a change in position of the valve in relation to said seat.

6. Device according to one of Claims 1 to 5, **characterized in that** the maximum rotational movement of the selector (1) is made according to an angle less than 360° about the axis (X-X).

7. Device according to one of Claims 1 to 6, **characterized in that** the reference points or markings (2) distributed around the rotation axis (X-X) of the selection means (1) are borne by a structure forming an external hood (6), preferably a hood made of a polymer.

8. Device according to Claim 7, **characterized in that** the reference points or markings (2) are situated on a dial (7) arranged or fixed about the axis (X-X), said dial (7) is preferably situated on the structure forming the external hood (6).

## Patentansprüche

1. Vakuumreglervorrichtung, die Folgendes aufweist:
- ein Wahlschaltermittel (1) für das Vakuumniveau, das durch das Bedienungspersonal manuell betätigt werden kann, um eine Drehachse (X-X) drehbeweglich ist und auf interne Vakuumregelungsmittel wirkt, um die Regelung des gewünschten Vakuumniveaus zu ermöglichen, und
- Anzeigermittel für das Vakuumniveau (P1, P2...; Pn; 2), die das Anzeigen eines Vakuumniveaus zu einem gegebenen Zeitpunkt für das Bedienungspersonal ermöglichen,
wobei das Wahlschaltermittel (1) durch das Bedienungspersonal zwischen mehreren winklig voneinander beabstandeten Stellungen (P1, P2...Pn) verschoben werden kann, wobei jede Winkelstellung (P1, P2...Pn) mindestens einem Wert eines auswählbaren Vakuumniveaus entspricht,
**dadurch gekennzeichnet, dass** jede Winkelstellung (P1, P2...Pn) durch eine Markierung oder eine Kennzeichnung (2), die um die Drehachse (X-X) des Wahlschaltermittels (1) verteilt sind, indiziert und markiert ist, derart, dass dem Bedienungspersonal das Vakuumniveau, das einer Winkelstellung (P1, P2...Pn) entspricht, in der das Wahlschaltermittel (1) zu einem gegebenen Zeitpunkt positioniert ist, direkt angezeigt wird, und **dadurch**, dass der drehbare Wahlschalter (1) oder beziehungsweise der Körper der Vorrichtung oder eine Struktur, die mit dem Körper verbunden ist, ein bewegliches Teil aufweist, das normalerweise durch ein Federmittel zurückgedrückt wird, und **dadurch**, dass das bewegliche Teil sich in jeder der indizierten Winkelstellungen (P1, P2...Pn) mindestens teilweise in eine Aussparung, die im Körper der Vorrichtung oder in einer mit dem Körper verbundenen Struktur eingerichtet ist, beziehungsweise mindestens in eine im drehbaren Wahlschalter (1) eingerichtete Aussparung einschiebt.

2. Vorrichtung nach Anspruch 1, **dadurch gekennzeichnet, dass** der Wahlschalter (1) für das Vakuumniveau ein Stellungsanzeigemittel (3, 4) aufweist, das das Anzeigen der Winkelstellung des Wahlschalters (1) um seine Achse (X-X) und im Verhältnis zu den Markierungen (2) ermöglicht.

3. Vorrichtung nach Anspruch 2, **dadurch gekennzeichnet, dass** das Stellungsanzeigemittel (3, 4) eine durch den drehbaren Wahlschalter (1) getragene Marke (3) oder ein im drehbaren Wahlschalter (1) eingerichtetes Lesefenster (4) ist.

4. Vorrichtung nach einem der Ansprüche 1 bis 3, **dadurch gekennzeichnet, dass** der drehbare Wahlschalter (1) aus Polymer besteht.

5. Vorrichtung nach einem der Ansprüche 1 bis 4, **dadurch gekennzeichnet, dass** die internen Vakuumregelungsmittel mindestens einen Sitz und ein Ventil aufweisen, und **dadurch**, dass die Verschiebung des Wahl schalters (1) von einer indizierten Winkelstellung in eine andere indizierte Winkelstellung (P1, P2...Pn) eine Veränderung der Positionierung des Ventils im Verhältnis zum Sitz bewirkt.

6. Vorrichtung nach einem der Ansprüche 1 bis 5, **dadurch gekennzeichnet, dass** die maximale Drehverschiebung des Wahlschalters (1) gemäß einem Winkel um die Achse (X-X) erfolgt, der kleiner als 360° ist.

7. Vorrichtung nach einem der Ansprüche 1 bis 6, **dadurch gekennzeichnet, dass** die Markierungen oder Kennzeichnungen (2), die um die Drehachse (X-X) des Wahlschaltermittels (1) verteilt sind, durch eine Struktur getragen werden, die eine Außenverkleidung (6), vorzugsweise eine Polymerverkleidung, bildet.

8. Vorrichtung nach Anspruch 7, **dadurch gekennzeichnet, dass** die Markierungen oder Kennzeichnungen (2) sich in einer Skala (7) befinden, die um eine Achse (X-X) eingerichtet oder befestigt ist, wobei die Skala (7) sich vorzugsweise auf der Struktur befindet, die die Außenverkleidung (6) bildet.
